**Europäisches Patentamt**

**European Patent Office**

**Office européen des brevets**

(19)

(11) Veröffentlichungsnummer : **0 055 859**
**B1**

(12) **EUROPÄISCHE PATENTSCHRIFT**

(45) Veröffentlichungstag der Patentschrift :
13.03.85

(21) Anmeldenummer : **81110835.6**

(22) Anmeldetag : **29.12.81**

(51) Int. Cl.⁴ : **A 61 B  5/14, A 61 M  5/34**

(54) **Blutentnahmevorrichtung.**

(30) Priorität : 30.12.80 DE 3049503

(43) Veröffentlichungstag der Anmeldung :
**14.07.82 Patentblatt 82/28**

(45) Bekanntmachung des Hinweises auf die Patenterteilung : **13.03.85 Patentblatt 85/11**

(84) Benannte Vertragsstaaten :
**AT BE CH FR GB LI LU NL SE**

(56) Entgegenhaltungen :
**CH-A-   303 837**
**CH-A-   590 661**
**DE-A- 2 028 662**
**GB-A- 1 036 000**
**US-A- 2 371 086**
**US-A- 2 828 742**
**US-A- 3 096 763**
**US-A- 3 503 386**

(73) Patentinhaber :  **Walter   Sarstedt   Kunststoff-
Spritzgusswerk**
**D-5223 Nümbrecht/Rommelsdorf (DE)**

(72) Erfinder : **Sarstedt, Walter**

**D-5223 Nümbrecht/Rommelsdorf (DE)**

(74) Vertreter : **Dipl.-Phys.Dr. Manitz Dipl.-Ing. Finsterwald
Dipl.-Ing. Grämkow Dipl.-Chem.Dr. Heyn
Dipl.-Phys.Rotermund
B.Sc. Morgan Robert-Koch-Strasse 1
D-8000 München 22 (DE)**

## Beschreibung

Die Erfindung betrifft eine Blutentnahmevorrichtung nach dem Oberbegriff des Anspruches 1.

Es ist bereits eine auch als Vorrichtung zum Entnehmen von Flüssigkeitsproben geeignete Injektionspritze bekannt (CH-A 590 661), bei der das vordere Ende eines Entnahmeröhrchens von einer durchstechbaren Membrane verschlossen ist. Auf das betreffende Ende des Entnahmeröhrchens ist eine Führungskappe aufgesetzt, deren vordere Stirnseite eine mit zwei Ausbuchtungen versehene Einsetzöffnung für eine die Kanüle tragende Führungshülse aufweist. Die Führungshülse besitzt zu den Ausbuchtungen komplementäre Rippen, so daß die die Kanüle tragende Führungshülse axial in die Führungskappe einschiebbar ist, bis sich die Rippen vollständig innerhalb der Führungskappe befinden. Während dieses Einsteckvorganges wird die Membran vollständig von dem hinteren Ende der Kanüle durchstochen. Anschließend kann dann die Führungshülse verdreht werden, wodurch eine bajonettverschlußartige Drehverriegelung bewirkt wird. Aufgrund der Axialführung während der Verschiebung der Führungshülse innerhalb der Führungskappe erfolgt während des Durchstechens der Membran keine Relativverdrehung zwischen dieser und der Kanüle. Dadurch ist es unmöglich, daß von der Membran kleine Stücke weggeschnitten werden.

Da die Passung zwischen der Führungskappe und der Führungshülse zwecks leichten Einschiebens der Führungshülse in die Führungskappe relativ locker ist, besteht eine gewisse Gefahr, daß sich die Drehverriegelung bei ungeschickter Handhabung der Spritze lockert und somit die Kanüle ungewollt aus dem Innern des Probenröhrchens herausrutschen kann. Um dem entgegenzuwirken, ist zwar beim Verdrehen eine Verklemmung vorgesehen, hierdurch ist es aber problematisch, wenn nicht gar unmöglich, das Entnahmeröhrchen von der Führungshülse zu trennen und durch ein neues zu ersetzen, solange die Nadel in der Vene steckt. Gerade dies aber ist bei Blutuntersuchungen häufig erforderlich, um mehrere Entnahmeröhrchen mit Blut zu füllen, ohne mehrmals eine neue Vene anstechen zu müssen.

Weiter ist schon eine bajonettverschlußartige Verbindung zwischen einem die Kanüle enthaltenden Zapfen und einer an der Vorderseite eines Röhrchens vorgesehenen Hülse bekannt (US-A 28 28 742), wobei jedoch an beiden Enden eines Längsschlitzes Erweiterungen vorgesehen sind, um eine schraubenartige Bewegung beim Befestigen des Ansatzes an der Hülse zu erzielen. Besondere Mittel zum Sichern des Bajonettverschlusses sind nicht vorgesehen, so daß ein unbeabsichtigtes Lösen des Ansatzes von der Hülse nur durch eine gewisse Schwergängigkeit der relativ zueinander beweglichen Elemente erzielt werden kann, was aber die obenerwähnten Nachteile nach sich zieht, wenn man unter Belassen der Nadel in einer Vene das Röhrchen abnehmen und durch ein neues ersetzen will. Bei der bekannten Vorrichtung ist zwar uber das vorn vorstehende Stück der Kanüle eine flexible Hülle gezogen, die sich beim Einsetzen des die Kanüle enthaltenden Ansatzes in die Hülse am Entnahmeröhrchen ausbaucht. Da sich die Hülle hierbei aber auch axial zusammen mit dem Ansatz in Richtung des Röhrchens verschiebt, kann so keine axiale Sicherungskraft für den Bajonettverschluß erzeugt werden, abgesehen davon, daß die flexible Hülle vor dem Gebrauch der Injektionsspritze entfernt wird.

Es ist zwar bereits bekannt (US-A 35 03 386) eine bajonettverschlußartige Drehverriegelung gegen unbeabsichtigtes Lösen zu sichern. Hierzu wird jedoch eine besondere Schraubendruckfeder benötigt.

Das Ziel der vorliegenden Erfindung besteht somit darin, eine Blutentnahmevorrichtung der eingangs genannten Gattung zu schaffen, welche bei lockerer Passung zwischen Ansatz und Führungshülse ohne Klemmung und ohne eine besondere Schraubendruckfeder, sondern nur unter Verwendung von bei Blutentnahmevorrichtungen üblichen Bauteilen eine einwandfreie Sicherung der Drehverriegelung gegen unbeabsichtigtes Lösen gewährleistet.

Zur Lösung dieser Aufgabe ist die Lehre des kennzeichnenden Teils des Anspruches 1 vorgesehen.

Erfindungsgemäß wird also zur Sicherung der Drehverriegelungsstellung ein bei Blutentnahmevorrichtungen zur sterilen Einhüllung des Kanülenendes üblicher dünner Schlauch herangezogen (z. B. DE-A 18 12 742 ; US-A 39 31 815), bei dessen Auswahl jedoch auf das Vorliegen der angesichts der lockeren Passung zur Sicherung der drehverriegelung erforderlichen Elastizität zu achten ist. Der Schlauch erfüllt erfindungsgemäß somit eine Doppelfunktion, indem er sowohl das Ende der Kanüle steril einhüllt als auch die für die Sicherung der Drehverriegelung erforderliche Axialspannkraft erzeugt. Eine besondere Spannfeder für die Sicherung der Drehverriegelung erübrigt sich somit. Auch der Verschlußstopfen kann so als dünne Membran ausgebildet werden, da die Nadel nach dem Durchstechen nicht mit starker Reibung in den Verschlußstopfen zu sitzen braucht. Die Führungshülse läßt sich somit leichtgängig auf den Ansatz aufsetzen und auch wieder von ihm abnehmen, so daß diese Vorgänge problemlos auch bei in der Vene steckender Nadel durchgeführt werden können. Gleichwohl ist ein sicherer Zusammenhalt der locker aneinander angeordneten Teile, nämlich der Führungshülse und des Ansatzes, gewährleistet. Die Erfindung verbindet somit einmal die leichte Trennbarkeit und Zusammenbringbarkeit des Ansatzes und der Führungshülse einerseits und deren sicheren Zusammenhalt in der Einstechposition andererseits.

Vorteilhafte Weiterbildungen der Erfindung sind durch die Unteransprüche gekennzeichnet.

Während die Ausführungsform nach Anspruch 3 die Drehverriegelung durch Andrücken der Nocken an die Flanken der Erweiterungen sichert, liegt bei dem Ausführungsbeispiel nach Anspruch 4 eine Rastvertiefung vor, in die die haltenocken durch den axial federnden Schlauch hineingedrückt werden, was zu einer besonders sicheren Halterung führt.

Durch die Ausführungsform nach Anspruch 5 wird das Einführen der Haltenocken in die Axialschlitze erleichtert.

Die Erfindung wird im folgenden beispielsweise anhand der Zeichnung beschrieben ; in dieser zeigt :

Figur 1 eine teilweise unterbrochene Seitenansicht einer erfindungsgemäßen Blutentnahmevorrichtung,

Figur 2 eine vergrößerte, teilweise geschnittene Seitenansicht des vorderen Teils dieser Vorrichtung,

Figur 3 eine teilweise geschnittene Seitenansicht ähnlich Fig. 2, die die Führungshülse und den Ansatz im zusammengeschobenen Zustand zeigt,

Figur 4 eine vergrößerte Teilseitenansicht einer anderen Ausbildung der Führungshülse,

Figur 5 eine Seitenansicht ähnlich Fig. 4 einer weiteren Ausführungsform und die

Figuren 6 und 7 zwei weiteren Ausführungsformen der Axialschlitze in Teilseitenansicht.

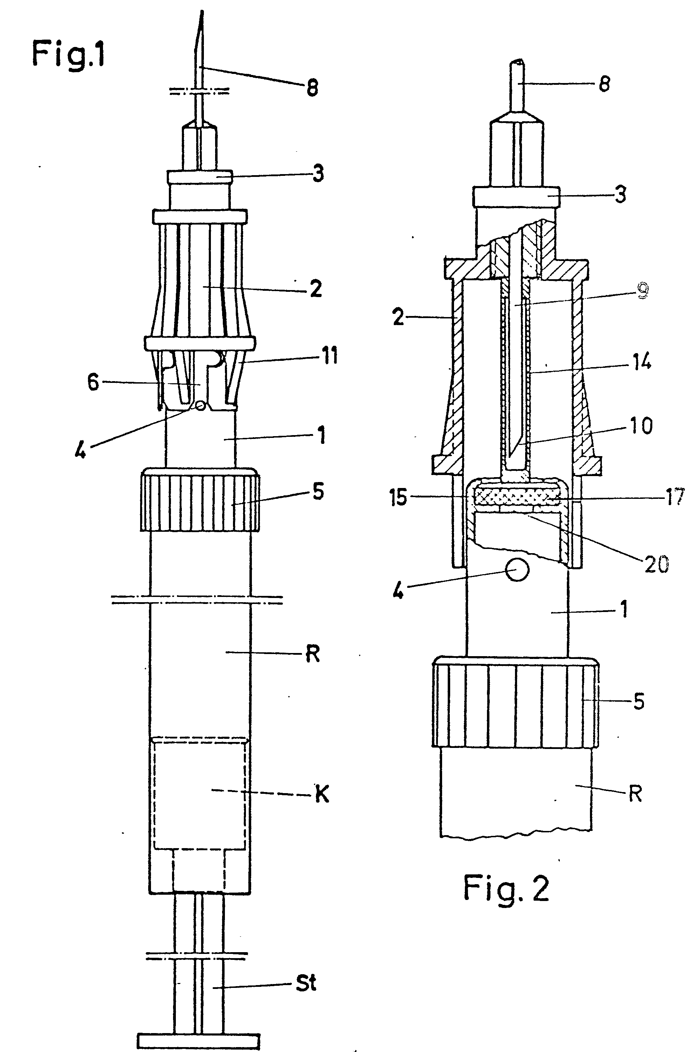

Die in den Fig. 1 bis 3 dargestellte Blutentnahmevorrichtung umfaßt ein zylindrisches Entnahmeröhrchen R, in dem ein gestrichelt angedeuteter Kolben K mit angesetzter Kolbenstange St luftdicht verschiebbar angeordnet ist. Das Entnahmeröhrchen R ist an seinem vorderen Ende durch eine Verschlußkappe 5 abgeschlossen, von der ein zylindrischer Ansatz 1 mit etwas geringerem Durchmesser in axialer Richtung vorsteht. Der Ansatz ist an seinem vorderen Ende durch einen durchstechbaren Verschlußstopfen 17 abgeschlossen, der auf einer mit einer Mittelbohrung 20 versehenen Stirnplatte des Ansatzes 1 aufliegt und von einem am vorderen Ende umgebördelten Kragen 15 gehalten ist.

Vom Ansatz 1 steht seitlich ein Haltenocken 4 vor, der einstückig mit der Wand des Ansatzes 1 ausgebildet ist.

Auf dem Ansatz 1 ist eine Führungshülse 2 axial verschiebbar und drehbar angeordnet, in deren vorderes, verengtes Ende ein Halter 3 mit einer eingegossenen Kanüle 8 eingeschraubt ist. Die Kanüle ist doppelendig und an beiden Enden angeschärft, d. h. mit Schneidkanten versehen. Das vordere Ende dient zur Einführung in eine Vene während das hintere als Kanülenende bezeichnete Teil 9 über den Halter 3 hinaus in die Führungshülse 2 hineinragt. Das hintere Kanülenende 9 ist kürzer als das in die Vene einzuführende vordere Ende.

Nach Fig. 2 ist auf das hintere Kanülenende 9 ein sackartiger Schauch 14 von solcher Länge aufgeschoben, daß die hintere Schneidkante 10 des hinteren Kanülenendes 9 bei gestrecktem Schlauch 14 noch nicht dessen Boden berührt.

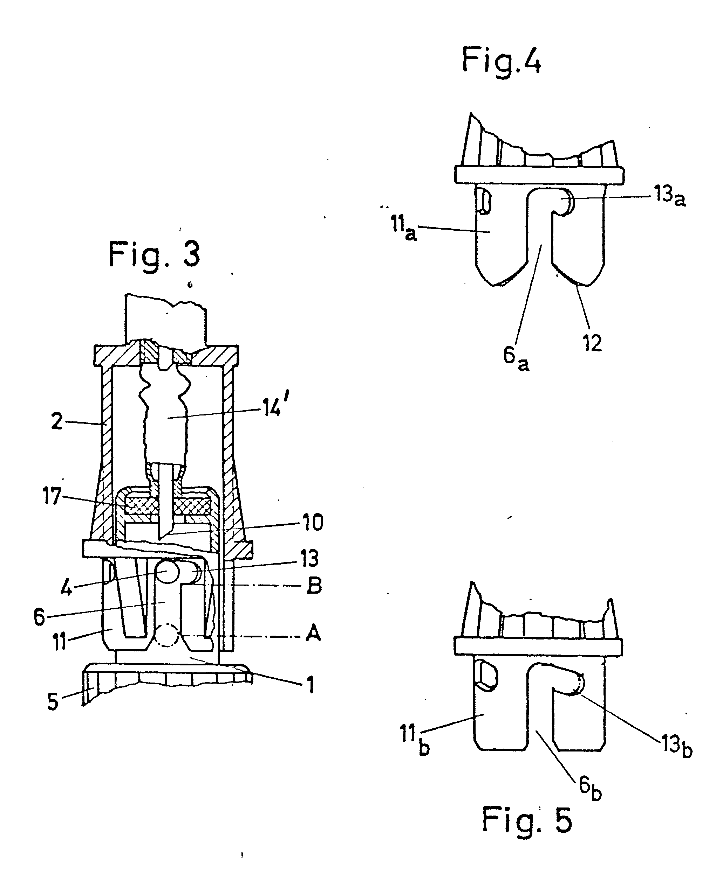

Im hinteren Bereich der Führungshülse 2, der aussen mit Längsrippen versehen sein kann, die ein leichteres Verschieben auf dem Ansatz 1 trotz guter Führung ermöglichen, sind über den Umfang verteilt Axialschlitze 6 vorgesehen, deren Breite etwas größer als die Stärke des Haltenockens 4 ist und welche gemäß Fig. 1 und 3 am vorderen Ende in eine seitliche Erweiterung 13 übergehen, die eine Fortsetzung der Axialschlitze im wesentlichen in Umfangsrichtung darstellt.

Bei Aufsetzen der Führungshülse 2 auf den Ansatz 1 wird der Haltenocken 4 in einen der über den Umfang verteilten Axialschlitze 6 eingeführt. Beim weiteren Aufschieben stößt zunächst der Boden des Schlauches 14 auf den Verschlußstopfen 17 auf, worauf die Schneidkante 10 des hinteren Kanülenendes 9 auf das verschlossene Ende des Schlauches 14 trifft. Nunmehr ist der Haltenocken 4 vollständig in den Axialschlitz 6 eingelaufen (strichpunktierte Darstellung A in Fig. 3), und es ist eine Axialführung der Führungshülse 2 relativ zum Ansatz 1 gewährleistet. Von jetzt an ist also eine Verdrehung der Führungshülse 2 und damit auch der Schneidkante 10 der Kanüle 8 gegenüber dem Ansatz 1 nicht mehr möglich. Ein weiteres Abwärtsschieben der Führungshülse 2 auf dem Ansatz 1 hat zur Folge, daß die Schneidkante 10 zunächst durch den Boden des Schlauches 14 und anschliessend durch den Verschlußstopfen 17 hindurchsticht.

Nach Fig. 3 sind die Länge der Axialschlitze 6 und die Anordnung des Haltenockens 4 so getroffen, daß der Haltenocken 4 bis zu einer Position B im Axialschlitz 6 geführt wird, in der die Schneidkante 10 der Kanüle 8 den Verschlußstopfen 17 bereits durchquert hat.

Sobald sich die Teile in der in Fig. 3 dargestellten Lage befinden, kann die Führungshülse 2 gegenüber dem Ansatz 1 auf der Verschlußkappe 5 zur axialen Verriegelung so verdreht werden, daß der Haltenocken 4 in die abgewinkelte kurze Erweiterung 13 einläuft.

Nach Fig. 3 ist der Schlauch 14 während des Aufschiebens der Führungshülse 2 auf den Ansatz 1 ziehharmonikaartig in die in Fig. 3 dargestellte Form 14' zusammengeschoben worden. Der Schlauch übt jetzt eine Axialkraft aus, die den Ansatz 1 aus der Führungshülse 2 herauszuschieben sucht. Das wird aber dadurch verhindert, daß der Haltenocken 4 in die Erweiterung 13 eingetreten ist. Zwischen der Führungshülse 2 und dem Ansatz 1 liegt also eine drehverriegelung vor, welche erst nach dem Durchstoßen der Schneidkante 10 durch den Boden des Schlauches 14 und des Verschlußstopfens 17 wirksam werden kann.

Nach Fig. 4 kann die Erweiterung 13a am Ende breiter sein, so daß der dort einlaufende Haltenocken 4 gegen eine unbeabsichtigte Rückdrehung gesichert ist, denn der ziehharmonikaartig zusammengedrückte Schlauch 14 übt eine Axialkraft aus, die die Führungshülse 2 vom Ansatz 1 wegzuschieben sich, wodurch der Haltenocken 4 in das breitere Ende der Erweiterung 13a hineingedrückt und die Verriegelung damit zusätzlich gesichert wird.

Der hintere Bereich 11a der Führungshülse 2 ist bei dieser Ausführungsform mit mehreren, über den Umfang verteilten Axialschlitzen 6a versehen, die an ihren Einlaufenden trichterförmige Erweiterungen 12 haben, die jeweils aneinander angrenzen.

Bei der in Fig. 5 dargestellten Ausführung ist der hintere Bereich 11b der Führungshülse 2 wieder mit mehreren, über den Umfang verteilten Axialschlitzen 6b versehen. Die an das Ende eines jeden Axialschlitzes 6b anschließende Erweiterung 13b ist dabei unter einem spitzen Winkel nach hinten abgewinkelt.

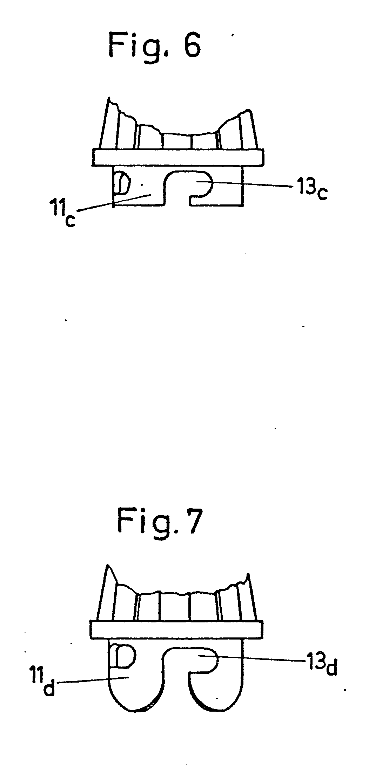

Die Fig. 6 und 7 zeigen Ausführungen mit einem ganz kurzen Axialschlitz im hinteren Bereich 11c der Führungshülse 2, an den sich dann die abgewinkelte kurze Erweiterung 13c bzw. 13d anschließt. Der Axialschlitz muß jedoch ausreichend lang sein, um beim Durchstoßen der Schneidkante 10 durch den Bodendes Schlauches 14 und dem Verschlußstopfen 17 eine Axialführung zwischen dem Ansatz 1 und der Führungshülse 2 zu gewährleisten. Bei dem Ausführungsbeispiel nach Fig. 7 weist der kurze Axialschlitz außerdem eine trichterförmige Erweiterung am hinteren Ende auf.

## Ansprüche

1. Blutentnahmevorrichtung, die ein zylindrisches Entnahmeröhrchen (R) mit einem darin luftdicht verschiebbaren Kolben (K) und einer vorderen, einen Ansatz (1) tragenden Verschlusskappe (5) sowie einem das Entnahmeröhrchen (R) verschließenden, durchstechbaren Verschlußstopfen (17) und eine an den Ansatz (1) ansetzbare rohrförmige Führungshülse (2) aufweist, die an ihrem vorderen Ende eine doppelendige, beiderseits mit einer Schneidkante versehene Kanüle (8) trägt, deren aus der Führungshülse (2) vorstehendes Ende zur Einführung in eine Vene vorgesehen ist, während ihr hinteres Ende so weit in die Führungshülse (2) hineinragt, daß es beim Ansetzen der Führungshülse (2) an das Entnahmeröhrchen (R) den Verschlußstopfen (17) durchsticht, wobei zwischen der Führungshülse (2) und dem Entnahmeröhrchen (R) wenigstens entlang eines Teils der Bewegung der hinteren Schneidkante (10) der Kanüle (8) durch das Material des Verschlußstopfens (17) eine Axialführung (6, 4) und nach vollständigem Durchstoßen des Verschlußstopfens (17) zwischen der an den Ansatz (1) angesetzten Führungshülse (2) und dem Ansatz (1) eine Drehverriegelung (13, 4) vorgesehen ist, bei der in Drehverriegelungsstellung eine die Drehverriegelung (13, 4) sichernde Axialspannkraft zwischen dem Ansatz (1) und der Führungshülse (2) vorliegt, dadurch gekennzeichnet, daß der von der Führungshülse (2) nach hinten ragende Teil (9) der Kanüle (8) von einem an seinem einen Ende durchstechbaren dünnen Schlauch (14) von solcher Elastizität eingehüllt ist, daß in der Drehverriegelungsstellung der harmonikaartig zusammengedrückte Schlauch (14) die die Drehverriegelung sichernde Axialspannkraft zwischen dem Ansatz (1) und der in lockerer Passung daraufsitzenden Führungshülse (2) bewirkt.

2. Blutentnahmevorrichtung nach Anspruch 1, dadurch gekennzeichnet, daß als Dreverriegelung am Ansatz (1) an dessen Umfang mindestens eine radial nach außen vorstehende Haltenocke (4) vorgesehen ist und in der außen auf dem Ansatz (1) sitzenden Führungshülse (2) Axialschlitze (6, 6a, 6b) vorgesehen sind, die an ihren vorderen Enden seitliche Erweiterungen (13, 13a, 13b, 13c, 13d) aufweisen.

3. Blutentnahmevorrichtung nach Anspruch 2, dadurch gekennzeichnet, daß die Erweiterungen (13, 13c, 13d) rechtwinklig zur Richtung der Axialspannkraft verlaufen.

4. Blutentnahmevorrichtung nach Anspruch 2, dadurch gekennzeichnet, daß die Erweiterungen (13a, 13b) sich von den Axialschlitzen (6a) weg etwas erweitern oder schräg von den Axialschlitzen (6b) weg verlaufen.

5. Blutentnahmevorrichtung nach einem der vorhergehenden Ansprüche, dadurch gekennzeichnet, daß die Axialschlitze (6) an ihren hinteren Enden trichterförmige Erweiterungen aufweisen.

## Claims

1. A blood extraction device comprising a cylindrical extraction tube (R), the cylindrical extraction tube having a piston (K) displaceable therein in airtight manner, a front closure cap (5) carrying a projection (1) and also a puncturable closure plug (17) which closes the extraction tube (R) ; and a tubular guide sleeve (2) which can be placed onto the projection (1) ; wherein the tubular guide sleeve carries at its front end a double ended cannula provided with a cutting edge at both ends ; wherein the end of the cannula which projects out of the guide sleeve (2) is provided for insertion into a vein whereas its rear end projects sufficiently far into the guide sleeve (2) that it punctures the closure plug (17) when the guide sleeve (2) is placed onto the extraction tube (R) ; wherein an axial guide (6, 4) is provided between the guide sleeve (2) and the extraction tube (3) for at least part of the movement of the rear cutting edge (10) of the cannula (8) through the material of the closure plug (17) ; and wherein a rotary locking arrangement (13, 4) is provided after complete penetration of the closure plug (17) between the guide sleeve (2) which is placed onto the projection (1) and the projection (1), with an axial bias force being present between the projection (1) and the guide sleeve (2) which secures the rotary locking arrangement (13, 4) in the rotated locked position, characterized in that the part (9) of the cannula (8) which projects rearwardly from the guide sleeve (2) is surrounded by a thin hose (14) which can be punctured at ist one end and which has an elasticity such that in the rotated locked position the hose (14'), which is compressed in harmonica-like manner, brings about the axial bias force between the projection (1) and the guide sleeve (2) which seats thereon with a loose fit, which secures the rotary locking arrangement.

2. A blood extraction device in accordance with claim 1, characterized in that at least one radially outwardly projecting retaining pin (4) is provided as the rotary locking arrangement on the projection (1) at its periphery and in that axial slots (6, 6a, 6b) are provided in the guide sleeve (2) which seats externally on the projection (1), with the axial slots having laterally broadened portions (13, 13a, 13b, 13c, 13d) at their front ends.

3. A blood extraction device in accordance with claim 2, characterized in that the broadened portions (13, 13c, 13d) extend at right angles to the direction of the axial bias force.

4. A blood extraction device in accordance with claim 2, characterized in that the broadened portions (13a, 13b) broaden somewhat away from the axial slots (6a), or extend obliquely away from the axial slots (6b).

5. A blood extraction device in accordance with one of the preceding claims, characterized in that the axial slots (6) have funnel-like broadened portions at their rear ends.

**Revendications**

1. Dispositif de prélèvement sanguin, qui présente un tube de prélèvement cylindrique (R) avec un piston (K) pouvant y coulisser de manière étanche à l'air, et un capuchon d'obturation avant (5) portant un appendice (1), ainsi qu'un bouchon d'obturation (17) perçable obturant le tube de prélèvement (R) et une douille de guidage tubulaire (2) pouvant être placée sur l'appendice (1) et portant à son extrémité avant une canule (8) à double extrémité, dotée de part et d'autre d'un tranchant, dont l'extrémité faisant saillie à partir de la douille de guidage (2) est prévue pour l'introduction dans une veine, tandis que son extrémité arrière pénètre sur une distance telle dans la douille de guidage (2) qu'elle perce lors de la mise en place de la douille de guidage (2) sur le tube de prélèvement (R), le bouchon d'obturation (17), tandis qu'entre la douille de guidage (2) et le tube de prélèvement (R), au moins sur une partie du déplacement du tranchant arrière (10) de la canule (8) à travers la matière du bouchon d'obturation (17), il est prévu un guidage axial (6, 4), et après la percée complète du bouchon d'obturation (17) entre la douille de guidage (2) placée sur l'appendice (1) et l'appendice (1), il est prévu un verrouillage par rotation (13, 4), dans lequel il existe en position de verrouillage par rotation, une force de serrage axiale assurant le verrouillage par rotation (13, 4) entre l'appendice (1) et la douille de guidage (2), caractérisé en ce que la partie (9) faisant saillie vers l'arrière à partir de la douille de guidage (2), de la canule (8) est enveloppée par une mince gaine (14) perçable à une de ses extrémités, avec une élasticité telle qu'en position de verrouillage par rotation, la gaine (14) comprimée en accordéon engendre la force de serrage axiale assurant le verrouillage par rotation entre l'appendice (1) et la douille de guidage (2) y reposant avec un ajustage lâche.

2. Dispositif de prélèvement sanguin suivant la revendication 1, caractérisé en ce qu'en tant que verrouillage par rotation, sur l'appendice (1), sur sa périphérie, est prévu au moins un mentonnet de retenue (4) faisant saillie radialement vers l'extérieur, et dans la douille de guidage (2) reposant extérieurement sur l'appendice (1) sont prévues des fentes axiales (6, 6a, 6b), qui présentent à leurs extrémités avant des élargissements latéraux (13, 13a, 13b, 13c, 13d).

3. Dispositif de prélèvement sanguin suivant la revendication 2, caractérisé en ce que les élargissements (13, 13c, 13d) s'étendent perpendiculairement à la direction de la force de serrage axiale.

4. Dispositif de prélèvement sanguin suivant la revendication 2, caractérisé en ce que les élargissements (13a, 13b) s'élargissent quelque peu à partir des fentes axiales (6a) ou s'étendent en oblique à partir des fentes axiales (6b).

5. Dispositif de prélèvement sanguin suivant l'une des revendications précédentes, caractérisé en ce que les fentes axiales (6) présentent des élargissements en entonnoir à leurs extrémités arrière.

Fig.1

Fig. 2

Fig.4

11ₐ  13ₐ

6ₐ  12

Fig. 3

2

14'

17

10

13

4  B

6

11  A

5  1

11ᵦ  13ᵦ

6ᵦ

Fig. 5

## Fig. 6

11c  13c

## Fig. 7

11d  13d